# DEMANDE DE BREVET EUROPEEN

(11) **EP 0 526 281 A1**
(43) Date de publication de la demande: **03.02.1993**
(21) Numéro de dépôt: 92401900.3
(22) Date de dépôt: 03.07.1992
(51) Int. Cl.: C07D 231/12, A01N 43/56, A61K 31/415

(54) **Procédé de synthèse de pyrazoles 1,4-disubstitués**

(30) Priorité: 24.07.1991 FR 9109331
(71) Demandeur: SOCIETE NATIONALE DES POUDRES ET EXPLOSIFS, F-75181 Paris Cédex 04 (FR)
(72) Inventeur: Alcaraz, Jean-Marie, F-91250 Saint Germain les Corbeil (FR); Lecacheur, Maryse, F-24100 Bergerac (FR); Robin, Yves, F-91710 Vert le Petit (FR)
(74) Mandataire: Pech, Bernard

(57) **Abrégé**

La présente invention est relative à un procédé de synthèse de pyrazoles 1,4-disubstitués. On fait réagir une hydrazine monosubstituée avec le produit de la réaction d'un acétal avec un sel d'halogénométhyliminium N-substitué, de préférence le chlorure de N,N-diméthyl chlorométhyliminium.

Les pyrazoles 1,4-disubstitués sont notamment d'importants intermédiaires de synthèse d'herbicides ou de médicaments.

## Description

La présente invention est relative à un nouveau procédé de synthèse de pyrazoles 1,4-disubstitués.

Les pyrazoles 1,4-disubstitués sont notamment d'importants intermédiaires de synthèse d'herbicides ou de médicaments.

Le brevet EP 366 328 décrit la synthèse de 4-mé- thylpyrazoles N-substitués par réaction du 2,3-dichl- loro-2-méthylpropanal avec une hydrazine monosubstituée.

Ce procédé est d'une part limité aux dérivés 4-méthyliques et d'autre part met en oeuvre des composés toxiques.

Le brevet DE 1 234 223 décrit la synthèse de pyrazoles 1,4-disubstitués par réaction d'une hydrazine monosubstituée avec le produit de la réaction d'une amine N-vinylique avec le produit de la réaction du phosgène avec le diméthylformamide (DMF). Le substituant en position 4 ne peut pas être un groupement aromatique, ce qui limite les applications. De plus, les amines N-vinyliques sont toxiques etd'un accès difficile, ce qui limite l'industrialisation d'un tel procédé, aussi bien en ce qui concerne la mise en oeuvre, qu'en ce qui concerne le coût et le traitement des effluents.

L'homme du métier est donc à la recherche d'un procédé industrialisable moins couteux que ceux précités, utilisant des matières premières facilement accessibles et non toxiques, qui puisse être généralisable à tous les pyrazoles 1,4-disubstitués.

La présente invention propose un tel procédé. Il est caractérisé en ce qu'on fait réagir une hydrazine monosubstituée avec le produit de la réaction d'un acétal avec un sel d'halogénométhyliminium N-substitué.

Les acétals sont des produits courants, bon marché, non toxiques, facilement accessibles, par exemple par réaction d'un alcool et d'un aldéhyde qui sont des matières premières de base de l'industrie chimique.

Le procédé selon l'invention est simple de mise en oeuvre, bon marché, généralisable à tous les pyrazoles 1,4-disubstitués, que l'on peut obtenir directement par une réaction "one-pot" utilisant 3 matières premières, sans isoler d'intermédiaire.

La nature chimique de ces matières premières et le procédé lui-même font que le traitement des effluents ne pose pas de problème particulier.

Selon l'invention on entend par "hydrazine monosubstituée" un composé obtenu par substitution d'un, et d'un seul, atome d'hydrogène de l'hydrazine NH₂-NH₂ par un radical organique.

Par ailleurs, le sel d'halogénométhyliminium est N-substitué, c'est-à-dire que l'atome d'azote quaternaire iminium 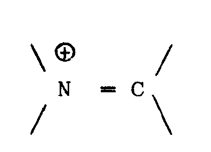
est relié à un (N-monosubstitué) ou deux (N,N-disubstitué) radicaux organiques, ce qui exclut

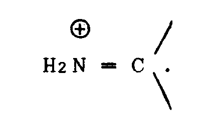

De façon préférée, selon l'invention, on obtient des pyrazoles 1,4-disubstitués de formule générale (III) 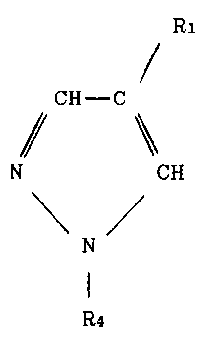
à partir d'acétals de formule générale (I) 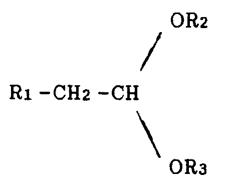
et d'hydrazines monosubstituées de formule générale (II)
R₄NH-NHz,
formules (I), (II) et (III) dans lesquelles :
- R₁ et R₄, identiques ou différents, représentent :
   . un radical aliphatique, de préférence alkyle Ci à C₂₄, linéaire ou ramifié, substitué ou non substitué,
   . un radical aryle, de préférence phényle ou naphtyle, substitué ou non substitué,
- R₂ et R₃, identiques ou différents, représentent un radical aliphatique, de préférence alkyle Ci à C₂₄, linéaire ou ramifié, substitué ou non substitué, ou bien encore R₂ et R₃ forment, conjointement avec les 2 atomes d'oxygène auxquels ils sont reliés et l'atome de carbone situé entre ces 2 atomes d'oxygène, un cycle comportant de préférence 3 à 49 atomes de carbone, substitué ou non substitué.

De façon préférée R₁ et R₄, identiques ou différents, représentent un radical alkyle Ci à C₁₂, de préférence Ci à C₄, ou un radical phényle, éventuellement substitué.

De façon également préférée, R₂ et R₃, identiques ou différents, représentent un radical alkyle Ci à C₁₂, de préférence Ci à C₄, ou bien encore R₂ et R₃ forment, conjointement avec les 2 atomes d'oxygène auxquels ils sont reliés et l'atome de carbone situé entre ces 2 atomes d'oxygène, un cycle comportant 3 à 7 atomes de carbone, par exemple un cycle 1,3-dioxolane.

Selon une variante préférée de l'invention, le sel d'halogénométhyliminium N-substitué est un halogénure d'halogénométhyliminium N-substitué, en général N,N-disubstitué , répondant de préférence à la formule générale (IV) : 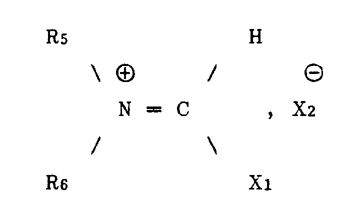
dans laquelle :
- X₁ et X₂, identiques ou différents, représentent un atome de chlore ou de brome,
- R₅ représente :
   . un radical aliphatique, de préférence alkyle Ci à C₂₄, linéaire ou ramifié, substitué ou non substitué,
   . un radical aryle, de préférence phényle ou naphtyle, substitué ou non substitué,
- R₆ représente :
   . l'hydrogène,
   . un radical aliphatique, de préférence alkyle Ci à C₂₄, linéaire ou ramifié, substitué ou non substitué,
   . un radical aryle, de préférence phényle ou naphtyle, substitué ou non substitué,
- ou bien encore R₅ et R₆ forment, conjointement avec l'atome d'azote auxquels ils sont reliés, un cycle comportant 3 à 48 atomes de carbone, de préférence 3 à 8 atomes de carbone, substitué ou non substitué.

Selon une variante particulièrement préférée, l'halogénure d'halogénométhyliminium N-substitué est un chlorure de chlorométhyliminium N-substitué, de préférence N,N-disubstitué, de préférence obtenu in situ dans le milieu réactionnel par réaction d'au moins un agent halogénant avec un amide N-substitué. Comme exemples d'agents halogénants, on peut
citer les agents bromants comme COBr₂ et les agents chlorants comme COCI₂, (COCI)₂, SOCI₂ et leurs mélanges. Le phosgène est particulièrement préféré.

La réaction entre l'acétal et le sel d'halogénomé- thyliminium N-substitué est en général mise en oeuvre en milieu solvant organique mais peut également se faire en l'absence de solvant. Comme exemples de solvants utilisables, on peut citer les hydrocarbures aliphatiques, éventuellement halogénés, les hydrocarbures aromatiques, éventuellement halogénés, par exemple ceux de type éther ou amide.

La température de réaction est en général comprise entre 20 et 150°C, de préférence voisine de 70°C.

Après réaction entre l'acétal et le sel d'halogéno- méthyliminium N-substitué, on ajoute, de préférence sans isoler le produit de cette réaction, l'hydrazine monosubstituée, pure, en solution aqueuse, ou en solution dans un solvant organique. On peut opérer de façon inverse c'est à dire ajouter dans l'hydrazine le produit de la réaction de l'acétal avec le sel d'halogé- nométhyliminium N-substitué.

Lorsque la réaction entre l'acétal et le sel d'halo- génométhyliminium N-substitué a lieu en présence d'un solvant organique, il n'est pas nécessaire d'éliminer celui-ci avant l'ajout de l'hydrazine monosubstituée, mais on peut néanmoins le faire.

Après l'ajout de l'hydrazine monosubstituée, on maintient en général la température entre 40 et 150°C, de préférence à environ 70°C.

La durée de chacune de ces 2 étapes du procédé selon l'invention est en général comprise entre 0,5 h et 4 h, de préférence voisine de 2 h.

Le mélange réactionnel est ensuite neutralisé par une solution aqueuse basique, par exemple de soude, potasse, carbonate alcalin, ou d'ammoniaque, pour permettre ensuite l'extraction du pyrazole 1,4-disubstitué formé par un solvant organique.

Après évaporation du solvant d'extraction, on peut récupérer le pyrazole 1,4-disubstitué pur à partir du produit brut par exemple par distillation sous vide partiel ou par recristallisation. De façon inattendue, on obtient un produit de plus grande pureté lorsqu'on traite au préalable le produit brut par une solution aqueuse alcaline concentrée (par exemple 30 %), à chaud, 40°C à 70°C par exemple. Un traitement de 1 h suffit en général. La teneur en DMF résiduel peut ainsi être inférieure à 0,5 % en poids, ce qui est particulièrement intéressant. D'autres sous-produits de synthèse sont également ainsi éliminés.

Le rapport molaire entre le sel d'halogénométhy- liminium N-substitué et l'acétal est en général compris entre 1,5 et 3, de préférence voisin de 2, et le rapport molaire entre l'hydrazine monosubstituée et l'acétal est en général compris entre 0,8 et 2, de préférence voisin de 1.

Lorsque le sel d'halogénométhyliminium N-substitué est un halogénure d'halogénométhyliminium N-substitué obtenu in situ dans le milieu réactionnel par réaction d'au moins un agent halogénant avec un amide N-substitué, le rapport molaire entre l'agent halogénant et l'acétal est en général compris entre 1,4 et 4, de préférence voisin de 2, le rapport molaire entre l'amide N-substitué et l'acétal est en général compris entre 0,8 et 3, de préférence voisin de 1, et le rapport molaire entre l'hydrazine monosubstituée et l'acétal est compris entre 0,8 et 2, de préférence voisin de 1.

Les exemples non limitatifs suivants illustrent l'invention et certains des avantages qu'elle procure.

### Exemple 1 Synthèse de 1,4-diméthylpyrazole à partir de propionaldéhyde diéthylacétal, de chlorure de N,N-diméthyl chlorométhyliminium et de monométhylhydrazine.

Dans un réacteur contenant 226,6 g (1,77 mol) de chlorure de N,N-diméthyl chlorométhyliminium en solution dans 400 ml de chloroforme, on introduit 116,2 g (0,88 mol) de propionaldéhyde diéthylacétal, à une température comprise entre 25°C et 55°C, puis on chauffe à 55°C pendant 2 h. On élimine le chloroforme sous vide partiel puis on introduit, à 50°C, 44,5 g (0,97 mol) de monométhylhydrazine en solution aqueuse à 30 %. On chauffe 2 h à 75°C. On refroidit le milieu réactionnel à 20°C, puis on ajoute une solution aqueuse de soude jusqu'à pH = 8. On ajoute alors 100 ml d'eau puis on procède à l'extraction du produit recherché par 3 fois 25 ml de chloroforme. On élimine le solvant d'extraction sous vide partiel puis on chauffe 1 h à 50°C en présence de soude à 30 %. On récupère le 1,4-diméthylpyrazole formé par décantation. Après séchage puis purification par distillation sous vide partiel, on obtient 56,6 g (0,59 mol) de 1,4-diméthylpyrazole pur. Le rendement en produit pur isolé est de 67 %.

### Exemple 2 Synthèse de 1,4-diméthylpyrazole à partir de 2-éthyl 1,3-dioxolane, de chlorure de N,N-diméthyl chlorométhyliminium et de monométhylhydrazine.

Dans un réacteur contenant 102,4 g (0,80 mol) de chlorure de N,N-diméthyl chlorométhyliminium en solution dans 400 ml de chloroforme, on introduit 40,8 g (0,4 mol) de 2-éthyl 1,3-dioxolane à une température comprise entre 50°C et 70°C, puis on chauffe 2 h à 75°C. On élimine le chloroforme sous vide partiel puis on introduit, à 50°C, 18,9 g (0,41 mol) de monométhylhydrazine en solution aqueuse à 30 %. On chauffe 2 h à 75°C. On refroidit le milieu réactionnel à 20°C puis on ajoute une solution aqueuse de soude jusqu'à pH = 8. On ajoute alors 40 ml d'eau puis on procède à l'extraction du produit recherché par 2 fois 20 ml de chloroforme. On élimine le solvant d'extraction puis on hydrolyse par chauffage 1 h à 50°C en présence de soude à 30 %. On récupère le 1,4-diméthylpyrazole par décantation, puis on le sèche et purifie par distillation sous vide partiel. Le rendement en produit pur isolé est de 72 %. Le rendement en produit formé, obtenu par dosage dans le produit brut, est égal à 90 %.

### Exemple 3 Synthèse de 1,4-diméthylpyrazole à partir de propionaldéhyde de diéthylacétal, de chlorure de N,N-diméthyl chlorométhyliminium généré in situ à partir de DMF et de phosgène, et de monométhylhydrazine.

Dans un réacteur contenant 310 ml de chloroforme, 29,2 g (0,4 mol) de DMF et 56,8 g (0,43 mol) de propionaldéhyde diéthylacétal, on introduit, à 10°C, 50 g (0,5 mol) de phosgène. On chauffe le milieu réactionnel à 30°C puis on introduit progressivement, en 1,5 h environ, à une température comprise entre 30°C et 50°C, 56 g (0,55 mol) de phosgène. On prolonge la réaction 0,5 h à 50°C. On introduit ensuite, à 30°C, 21,7 g (0,47 mol) de monométhylhydrazine, en solution aqueuse à 30 %. On laisse réagir 2 h à 75°C. On laisse refroidir puis on récupère la phase aqueuse après décantation. On neutralise cette phase aqueuse par une solution aqueuse de soude à 30 % jusqu'à pH = 7. On extrait la phase aqueuse par 3 fois 75 ml de chloroforme, puis on concentre les phases organiques pour obtenir 70,5 g de produit brut contenant 30,3 g de 1,4-diméthylpyrazole pur (rendement 73 %) et dont la teneur en DMF résiduel est inférieure à 0,3 %.

### Exemple 4 Synthèse de 1,4-diméthylpyrazole à partir de 2-éthyl 1,3-dioxolane, de chlorure de N-méthyl N-phényl chlorométhyliminium généré in situ à partir de phosgène et de N-méthylformanilide, et de monométhylhydrazine.

Dans un réacteur contenant 80 ml de toluène, 27 g (0,20 mol) de N-méthylformanilide et 20,5 g (0,20 mol) de 2-éthyl 1,3-dioxolane, on introduit, à une température de 70°C, 50 g (0,50 mol) de phosgène. On laisse réagir pendant 3 h à 70°C. On refroidit à 50°C, puis on introduit, à une température comprise entre 50°C et 75°C, 9,2 g (0,20 mol) de monométhylhydrazine en solution aqueuse à 30 %. On chauffe 2 h à 75°C. On refroidit à 20°C puis on neutralise jusqu'à pH = 8 avec une solution aqueuse de soude à 30 %. On ajoute 20 ml d'eau puis on récupère par décantation la phase organique qui contient 13 g de 1,4-diméthylpyrazole pur (rendement 70 %).

### Exemple 5 Synthèse de 1-phényl 4-méthylpyrazole à partir de 2-éthyl 1,3-dioxolane, de chlorure de N,N-diméthyl chlorométhyliminium et de monophénylhydrazine.

Dans un réacteur contenant 67,37 g (0,526 mol) de chlorure de N,N-diméthyl chlorométhyliminium en solution dans 175 ml de chloroforme, on introduit, à une température comprise entre 45°C et 60°C, 25 g (0,25 mol) de 2-éthyl 1,3-dioxolane. On chauffe 1 h à 60°C. On élimine le chloroforme sous vide partiel puis on introduit, à 50°C, 30 g (0,267 mol) de monophénylhydrazine en solution chloroformique à 30 %. On chauffe 2 h à 70°C. On refroidit à 20°C puis on ajoute une solution aqueuse de soude jusqu'à pH = 8. On ajoute alors 25 ml d'eau puis on procède à l'extraction du produit recherché par 2 fois 20 ml de chloroforme. On élimine le solvant d'extraction puis on hydrolyse par chauffage 1 h à 60°C en présence de soude aqueuse à 30 %. On récupère par décantation le 1- phényl 4-méthylpyrazole brut, identifié par spectrométries RMN ¹H et de masse. Un dosage réalisé sur le produit brut montre que l'on a formé le 1-phényl 4-méthylpyrazole avec un rendement de 65 %.

### Exemple 6 Synthèse de 1-méthyl 4-phénylpyrazole à partir de 2-benzyl 1,3-dioxolane, de chlorure de N,N-diméthyl chlorométhyliminium et de monométhylhydrazine.

Dans un réacteur contenant 57,6 g 10,45 mol) de chlorure de N,N-diméthyl chlorométhyliminium en solution dans 150 ml de chloroforme, on introduit, à une température comprise entre 50°C et 65°C, 32,8 g (0,20 mol) de 2-benzyl 1,3-dioxolane, puis on chauffe 1 h à 65°C. On élimine le chloroforme sous vide partiel puis on introduit, à 50°C, 11,5 g (0,25 mol) de monométhylhydrazine en solution aqueuse à 30 %. On chauffe 2 h à 70°C. On refroidit à 20°C puis on neutralise jusqu'à pH = 8 avec une solution aqueuse de soude à 30 %. On ajoute alors 10 ml d'eau puis on procède à l'extraction du produit recherché par 2 fois 15 ml de chlorure de méthylène. On élimine le solvant d'extraction. On obtient ainsi un produit brut que l'on a purifié par recristallisation dans l'éther isopropyli- que. Le produit purifié, du 1-méthyl 4-phénylpyrazole identifié par spectrométries RMN ¹H et de masse, a un point de fusion de 103,6°C. Le rendement en 1-méthyl 4-phénylpyrazole formé, déterminé par dosage sur le produit brut, est de 46 %.

## Revendications

1. Procédé de synthèse de pyrazoles 1,4-disubstitués caractérisé en ce qu'on fait réagir une hydrazine monosubstituée avec le produit de la réaction d'un acétal avec un sel d'halogénométhylimi- nium N-substitué.

2. Procédé de synthèse selon la revendication 1, caractérisé en ce que l'acétal répond à la formule générale (I) 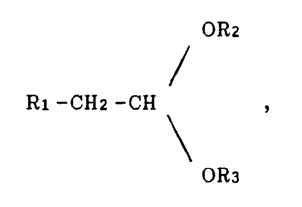 l'hydrazine monosubstituée à la formule générale (II) R₄NH-NH₂ et les pyrazoles 1,4-disubstitués obtenus à la formule générale (III) 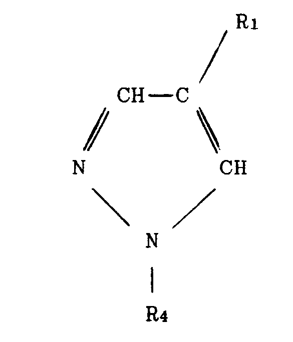
formules dans lesquelles :
- R₁ et R₄, identiques ou différents, représentent :
. un radical aliphatique, de préférence alkyle Ci à C₂₄, linéaire ou ramifié, substitué ou non substitué,
. un radical aryle, de préférence phényle ou naphtyle, substitué ou non substitué,
- R₂ et R₃, identiques ou différents, représentent un radical aliphatique, de préférence alkyle Ci à C₂₄, linéaire ou ramifié, substitué ou non substitué, ou bien encore R₂ et R₃ forment, conjointement avec les 2 atomes d'oxygène auxquels ils sont reliés et l'atome de carbone situé entre ces 2 atomes d'oxygène, un cycle comportant de préférence 3 à 49 atomes de carbone, substitué ou non substitué.

3. Procédé de synthèse de pyrazoles 1, 4-disubstitués selon la revendication 2, caractérisé en ce que R₁ et R₄, identiques ou différents, représentent un radical alkyle Ci à C₁₂, de préférence Ci à C₄, ou un radical phényle éventuellement substitué.

4. Procédé de synthèse de pyrazoles 1,4-disubstitués selon l'une quelconque des revendications 2 et 3, caractérisé en ce que R₂ et R₃, identiques ou différents, représentent un radical alkyle Ci à C₁₂, de préférence Ci à C₄, ou bien encore R₂ et R₃ forment conjointement avec les 2 atomes d'oxygène auxquels ils sont reliés et l'atome de carbone situé entre ces 2 atomes d'oxygène, un cycle comportant 3 à 7 atomes de carbone.

5. Procédé de synthèse de pyrazoles 1,4-disubstitués selon l'une quelconque des revendications précédentes, caractérisé en ce que le sel d'halo- génométhyliminium N-substitué est un halogénure d'halogénométhyliminium N-substitué.

6. Procédé de synthèse de pyrazoles 1,4-disubstitués selon la revendication 5, caractérisé en ce que l'halogénure d'halogénométhyliminium N-substitué répond à la formule générale (IV) : 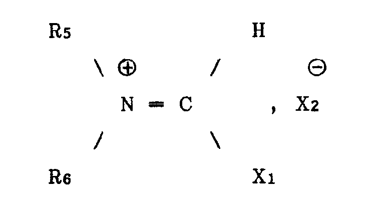
dans laquelle :
- X₁ et X₂, identiques ou différents, représentent un atome de chlore ou de brome,
- R₅ représente :
. un radical aliphatique, de préférence alkyle Ci à C₂₄, linéaire ou ramifié, substitué ou non substitué,
. un radical aryle, de préférence phényle ou naphtyle, substitué ou non substitué,
- R₆ représente :
. l'hydrogène,
. un radical aliphatique, de préférence alkyle Ci à C₂₄, linéaire ou ramifié, substitué ou non substitué,
. un radical aryle, de préférence phényle ou naphtyle, substitué ou non substitué,
- ou bien encore R₅ et R₆ forment, conjointement avec l'atome d'azote auxquels ils sont reliés, un cycle comportant 3 à 48 atomes de carbone, de préférence 3 à 8 atomes de carbone, substitué ou non substitué.

7. Procédé de synthèse de pyrazoles 1,4-disubstitués selon l'une quelconque des revendications 5 et 6, caractérisé en ce que l'halogénure d'halogé- nométhyliminium N-substitué est un chlorure de chlorométhyliminium N-substitué, de préférence N,N-disubstitué.

8. Procédé de synthèse de pyrazoles 1,4-disubstitués selon l'une quelconque des revendications 5 à 7, caractérisé en ce que l'halogénure d'halogé- nométhyliminium N-substitué est obtenu in situ dans le milieu réactionnel par réaction d'au moins un agent halogénant avec un amide N-substitué.

9. Procédé de synthèse de pyrazoles 1,4-disubstitués selon la revendication 8, caractérisé en ce que l'agent halogénant est un agent chlorant choisi dans le groupe constitué par COCI₂, (COCI)₂, SOCI₂ et leurs mélanges, de préférence COCI₂.

10. Procédé de synthèse selon l'une quelconque des revendications précédentes, caractérisé en ce que la réaction de l'acétal avec le sel d'halogéno- méthyliminium N-substitué est mise en oeuvre dans un solvant organique, à une température comprise entre 20 et 150°C.

11. Procédé de synthèse selon l'une quelconque des revendications 1 à 7 et 10, caractérisé en ce que le rapport molaire entre le sel d'halogénométhy- liminium N-substitué et l'acétal est compris entre 1,5 et 3, et le rapport molaire entre l'hydrazine monosubstituée et l'acétal est compris entre 0,8 et 2.

12. Procédé de synthèse selon l'une quelconque des revendications 8 et 9, caractérisé en ce que le rapport molaire entre l'agent halogénant et l'acétal est compris entre 1,4 et 4, le rapport molaire entre l'amide N-substitué et l'acétal est compris entre 0,8 et 3 et le rapport molaire entre l'hydrazine monosubstituée et l'acétal est compris entre 0,8 et 2.

13. Procédé de synthèse de pyrazoles 1,4-disubstitués selon l'une quelconque des revendications précédentes caractérisé en ce qu'après la réaction de l'hydrazine monosubstituée avec le produit de la réaction de l'acétal avec le sel d'halo- génométhyliminium N-substitué, on neutralise le milieu avec une solution aqueuse basique, on extrait le pyrazole 1,4-disubstitué par un solvant organique, on élimine le solvant d'extraction puis on purifie le produit brut obtenu.

14. Procédé de synthèse de pyrazoles 1,4-disubstitués selon la revendication 13, caractérisé en ce qu'on traite le produit brut obtenu par une solution aqueuse alcaline concentrée, à une température comprise entre 40°C et 70°C.
